# EUROPEAN PATENT SPECIFICATION

(11) **EP 4 302 789 B1**
(45) Date of publication and mention of the grant of the patent: **22.07.2026**
(21) Application number: 23195144.3
(22) Date of filing: 28.03.2013
(51) Int. Cl.: A01N 31/08, A01N 31/16, A01N 43/54, A01N 47/44, A61L 29/08, A61L 29/14

(54) **CATHETER ASSEMBLY**
KATHETERBAUGRUPP
ENSEMBLE DE CATHÉTER

(30) Priority: 03.04.2012 US 201213438559
(43) Date of publication of application: 10.01.2024
(62) Divisional of application: 13716643.5
(73) Proprietor: Becton, Dickinson and Company, Franklin Lakes, NJ 07417-1880 (US)
(72) Inventor: BURKHOLZ, Jonathan Karl, Salt Lake City, 84124 (US); HOANG, Minh Quang, Sandy, 84093 (US)
(74) Representative: dompatent

(56) References cited:
- WO-A2-02/051464
- WO-A2-2011/034675
- US-A- 4 925 668
- US-A1- 2010 204 648

## Description

### BACKGROUND OF THE INVENTION

This disclosure relates generally to antimicrobial materials and methods for applying the materials to various surfaces of a medical device. In particular, this disclosure discusses an antimicrobial coating material comprising one or more alcohol-based carrier solvents, a lubricant, and a biocidal agent, wherein the one or more carrier solvents assist in carrying and delivering the biocidal agent to the various surfaces of the medical device, and wherein the lubricant reduces friction between various components of the medical device during assembly. Moreover, this disclosure discusses a method of assembling a medical device, wherein the physical properties of the antimicrobial material assists in the assembly process. The claimed invention is directed towards a catheter assembly.

In the fields of medicine and health care, a patient's skin may be punctured in a variety of manners and for a variety of reasons. For example, a cannula or an intravenous ("IV") catheter is forced through the patient's skin into an interior space, such as the patient's vasculature. In this example, the cannula or IV catheter can be used for infusing fluid (e.g., saline solution, medicaments, and/or total parenteral nutrition) into the patient, withdrawing fluids (e.g., blood) from the patient, and/or monitoring various parameters of the patient's vascular system.

Bacteria and other harmful microbes are commonly introduced into a patient as a result of accessing the vasculature of a patient via a medical device. In some instances, harmful microbes are introduced at a site where the patient's skin was punctured. In other instances, microbes are present within fluid pathways of a medical device prior to accessing the patient's vasculature with the contaminated medical device. Further still, in some instances a harmful microbe is introduced into the vasculature of a patient by introducing fluids and medicaments to the patient via the inserted medical device, such as by a syringe, a needle or an IV fluid bag. Thus, infusion therapy techniques and procedures increase the likelihood of infection in the patient. Indeed, it is estimated that each year hundreds of thousands of patients in the United States alone develop some form of bloodstream infection that is caused by pathogens that were communicated to the patient through or because of an IV catheter or another IV access device, such as a hypodermic needle.

Often, these catheter-related bloodstream infections cause patient illness and, in some cases, death. Furthermore, because some infections are caused by bacterial strains (e.g., Methicillin-resistant *Staphylococcus aureus* ("MRSA") and Vancomycin-resistant *Enterococci* ("VRE")) that are resistant to antibiotics, such infections can be hard to treat and may be becoming more prevalent. Additionally, because patients that have a bloodstream infection may require additional medical treatment, catheter-related bloodstream infections may also be associated with increased medical costs.

In an attempt to limit bloodstream infections (i.e., catheter-related infections) in hospital, outpatient, home care, and other health care settings, many have implemented sanitary techniques. For example, many health care providers have placed a strong emphasis on wearing gloves, cleaning hands, cleaning the insertion site on patient's skin before the catheter or other sharp medical device punctures the skin, cleaning the catheter site after the puncture, and using sterilize medical instruments.

While hands, skin, medical instruments, and other surfaces in health care settings are cleaned in a variety of methods, often cleansers with one or more antimicrobial agents are used to clean such surfaces. However, such cleansers are not without their shortcomings. For example, many cleansers are ineffective against some common types of microbes. For instance, as mentioned above, some pathogens, such as MRSA and VRE, have developed a resistance to certain antimicrobial agents. Further, microbes located within the medical device are inaccessible to care providers and therefore are incapable of being sterilized during an infusion therapy or technique.
WO 2011/ 034675 A2 discloses a lubricious antiseptic coating material containing various solvents to achieve mutual miscibility and provide a generally homogenous product.
US 4 925 668 A2 discloses a substantially hydrophilic polymeric medical article having a coating of chlorhexidine and a silicone on its surface and which may have chlorhexidine bulk distributed throughout the polymer.
WO 02/ 051464 A2 discloses medical devices treated with a solution comprising one or more solvents and a combination of chlorhexidine free base and a water-soluble chlorhexidine salt in a weight/ weight ratio of between about 1:1 to about 1:5, preferably about 1:1.
US 2010/ 204648 A1 discloses a flushable catheter assembly having features to enable selective activation of fluid flow through the catheter assembly.

Thus, although techniques currently exist to minimize or eliminate BSIs in patient, challenges still exist. Accordingly, it would be an improvement in the art to augment, or even replace current techniques with new techniques and materials. Such techniques, materials and methods are provided herein.

### BRIEF SUMMARY OF THE INVENTION

The present application relates to systems and methods for applying an antimicrobial composition or coating material to a medical device. In particular, the present disclosure provides a method of assembling a medical device, wherein various components of a novel antimicrobial composition aid in the assembly of the medical device and provide an antimicrobial coating on much of the internal geometry of the medical device. In some instances, the physical properties of the novel antimicrobial composition further act as an adhesive to maintain the assembled relationship of various components of the medical device. Specifically, the present invention relates to a catheter assembly having a catheter adapter operably coupled to a catheter, wherein the catheter adapter and catheter each comprise various internal sufaces and geometries, said catheter assembly being obtainable from a method comprising the steps of:
providing a catheter assembly having an internal lumen;
providing a septum having an outer surface, wherein the outer diameter of the septum is greater than an opening of the catheter adapter;
coating the outer surface of the septum with an excess of an antimicrobial composition comprising:
   a non-alcohol, biocidal agent;
   a lubricant; and
   a carrier solvent comprising a non-alcohol based solvent in which the biocidal agent and lubricant are dissolved, and which readily undergoes evaporation at ambient conditions; inserting the coated septum into the internal lumen of the catheter adapter, wherein the septum compresses inwardly during insertion of the septum through the opening of the catheter adapter;
   distributing the excess antimicrobial composition to the internal lumen by advancing and seating the coated septum within the internal lumen, such that it is seated within a channel or groove formed on the inner surface of the catheter adapter;
   further distributirig the excess antimicrobial composition within the internal lumen and forcing the excess antimicrobial composition between opposing surfaces of components within the catheter assembly by blowing air through the internal lumen and the seated septum; and
   blowing additional air through the internal lumen and the seated septum to evaporate the carrier solvent of the antimicrobial composition thereby immobilizing the lubricant and the non-alcohol, biocidal agent within the internal lumen.
Preferred embodiments are evident from the subject-matter of the dependent claims.

The present invention employs an antimicrobial composition comprising a non-alcohol, biocidal agent, a lubricant, and a carrier solvent comprising a non-alcohol in which the biocidal agents and the lubricant are soluble. The biocidal agents may include any non-alcohol, biocidal agents or compound which is effective against pathogens. In some instances, the non-alcohol, biocidal agent is selected from a group consisting of phenol, quaternary ammonium, guanidine, pyridinium, benzalkonium, centrimide, benzethonium chloride, cetylpyridinium chloride, dequalium acetate, dequalinium chloride, chloroxylenol, chlorhexidine, chlorhexidine acetate, chlorhexidine hydrochloride, triclosan, chlorhexidine dihydrochloride, and combinations thereof.

The lubricant of the antimicrobial composition may include any lubricious material which is compatible with the teachings of the present invention. In some instances, the antimicrobial composition includes a lubricant having a low viscosity, such as a low viscosity siloxane. In other instances, the antimicrobial composition includes a lubricant having a surface tension of about 20mN/ m.

The carrier solvent of the antimicrobial compositions of the present technology in genral may include any alcohol-based solvent material which is capable of dissolving the lubricant and biocidal agents of the antimicrobial composition in accordance with the teachings of the present invention. In this context of the present technology in general, in some instances, the antimicrobial composition includes a carrier solvent comprising an alcohol selected from the group consisting of ethanol, isopropanol, propanol, butanol, and combinations thereof. In this context, in other instances, the antimicrobial composition includes a carrier solvent consisting of lower alcohols having from about 1 carbon atom to about 6 carbon atoms. However, in the invention as claimed the antimicrobial composition includes a carrier solvent comprising a non-alcohol based solvent in which the biocidal agent and lubricant are dissolved, and which readily undergoes evaporation at ambient conditions.

The claimed invention is obtainable by a method for assembling a medical device, wherein an antimicrobial composition assists in assembling the medical device, and wherein the individual components of the antimicrobial composition provide desired benefits to the assembly process and to the final, assembled medical device product. The claimed invention is a catheter assembly having a catheter adapter operably coupled to a catheter, wherein the catheter adapter and the catheter each comprise various internal surfaces and geometries as reflected in the claims.

For example, in some instances the lubricant of the antimicrobial composition reduces friction between various components of the medical device during assembly. Further, the carrier solvent dissolves the biocidal agents and assists in distributing the biocidal agent to various internal geometries of the medical device during assembly of the medical device. Once coated, the carrier solvent is evaporated thereby immobilizing the biocidal agent on the internal geometries of the medical device. The immobilized biocidal agent prevents bacterial growth or colonization on the coated, internal geometries of the medical device. In some implementations, a thin layer of biocidal agent remains interposed between various components of the medical device, wherein the tacky nature of the biocidal agent acts as an adhesive between the various components.

### BRIEF DESCRIPTION OF THE SEVERAL VIEWS OF THE DRAWINGS

In order that the manner in which the above-recited and other features and advantages of the invention are obtained and will be readily understood, a more particular description of the invention briefly described above will be rendered by reference to specific embodiments thereof, which are depicted in the appended Figures. Understanding that these Figures depict only typical embodiments of the invention and are not, therefore, to be considered to be limiting of its scope, the invention will be described and explained with additional specificity and detail through the use of the accompanying Figures in which:
Figure 1 illustrates a flow chart showing a method for applying an antimicrobial composition to internal structures and geometries of a medical device in accordance with a representative embodiment of the present technology in general.
Figure 2 illustrates a flow chart showing a method for applying an antimicrobial composition to internal structures and geometries of a medical device via coating an internal component of the medical device prior to assembly of the medical device in accordance with a representative embodiment of the present invention.
Figure 3 illustrates a medical device having an internal component prior to assembly of the medical device in accordance with a representative embodiment of the present invention.
Figure 4 illustrates a medical device having an internal component coated with an antimicrobial composition prior to assembly of the medical device in accordance with a representative embodiment of the present invention.
Figure 5 illustrates a medical device during assembly in accordance with a representative embodiment of the present invention.
Figure 6 illustrates a medical device following assembly and evaporation of an antimicrobial coating applied to various internal structures and geometries of the medical device in accordance with a representative embodiment of the present invention.

### DETAILED DESCRIPTION OF THE INVENTION

In order to provide a thorough understanding of the invention, the following description discusses specific details. The skilled artisan, however, would understand that the invention can be practiced without employing these specific details. Indeed, the invention can be modified in any suitable manner and can be used in conjunction with any suitable chemical, apparatus, and technique conventionally used in the industry. Thus, the following more detailed description of the embodiments of the invention is not intended to be limiting in scope, but is merely representative of some presently preferred embodiments.
Additionally, while the following discussion focuses on using the invention in health care settings, the antimicrobial composition may be used in any suitable setting.
Hereinbelow, frequent reference is made to a "medical device". The medical device of the invention as claimed is a catheter assembly having a catheter adapter operably coupled to a catheter, wherein the catheter adapter and catheter each comprise various internal surfaces and geometries as reflected in the present claims.

Generally, this application discusses an antimicrobial composition that is effective at killing and preventing the growth of a wide range of pathogens. As used herein the terms pathogen, pathogens and bacteria may include any potentially infectious microorganism, bacteria (e.g., undulating bacteria, gram-negative bacteria, gram-positive bacteria, aerobic bacteria, anaerobic bacteria, mycobacteria, spriochetes, *Staphylococcus epidermis, Staphylococcus aureus, Escerchia coli, Proteus vulgaris, Streptococcus faecalis, Klebsiella, Enterobacter aerogenes, Proteus mirabilis,* and the like), fungi (e.g., fungal spores, *Aspergillus niger, Aspergillus flavus, Rhizopus nigricans, Cladosporium herbarium, Epidermophyton Floccosum, Trichophyton mentagrophytes, Histoplasma capsulatum,* and the like), yeasts (e.g., *Saccharomyces cerevisiae, Candida albicans,* and the like), virus, and/or other potentially hazardous microbes. Additionally, in some presently preferred embodiments, the described antimicrobial composition preferably dries to leave a tacky residue. As used herein, the term tacky residue may connote a gummy, sticky, adhesive, and/or gluey deposit.

The antimicrobial composition may comprise any suitable ingredient that allows it to kill a wide range of pathogens, dry with leaving a tacky residue, and be suitable for dermal use on humans and/or use on medical devices. The antimicrobial composition employed in the invention as claimed comprises a non-alcohol biocidal agent, a lubricant, and a carrier solvent comprising a non-alcohol based solvent capable of dissolving the biocidal agent and lubricant. To provide a better understanding of the antimicrobial composition, the various components of the composition are described below in more detail.

The biocidal agent may comprise any chemical, besides alcohol, that is suitable for use on human skin and which kills and/or inhibits/prevents the propagation of potentially infectious pathogens. In some embodiments, the first biocidal agent comprises a salt of chlorhexidine, such as chlorhexidine gluconate. In other embodiments, the biocidal agent comprises physical properties whereby the agent leaves a sticky residue upon drying.

Chlorhexidine gluconate may have several characteristics that allow it to be an effective biocidal agent. In one example, chlorhexidine gluconate is effective at killing and inhibiting the growth of a wide variety of pathogens that are common to healthcare settings. In another example, when chlorhexidine gluconate in the antimicrobial composition dries, the chlorhexidine gluconate forms a tacky deposit on the surface to which it was applied. This tacky residue may remain on the surface for a period of time and, thereby, provide the surface with a residual biocidal effect. This tacky residue may further act as an adhesive or binding agent between two or more interfacing surfaces, where binding between the surfaces is desirable.

The antimicrobial composition may comprise any suitable concentration of biocidal agent that allows the composition to effectively kill or prevent pathogen proliferation while being safe for intravenous exposure. In some embodiments, an antimicrobial composition is provided which includes a chlorhexidine gluconate biocidal agent comprising from about 0.01% to about 1.0% of the total weight of the composition. In other embodiments, an antimicrobial composition is provided which includes a chlorhexidine gluconate biocidal agent comprising from about 0.01% to about 0.5% of the composition, by weight of the composition. In still other embodiments, an antimicrobial composition is provided which includes chlorhexidine gluconate as a biocidal agent from about 0.5% to about 2% of the composition, by weight of the composition.

The antimicrobial composition may further comprise other non-alcohol biocidal agents that allow the composition to effectively kill or prevent pathogen proliferation while being safe for intravenous exposure. Non-limiting examples of suitable biocidal agents include phenol, quaternary ammonium, guanidine, pyridinium, benzalkonium, centrimide, benzethonium chloride, cetylpyridinium chloride, dequalium acetate, dequalinium chloride, hexetidine, chloroxylenol, chlorhexidine, chlorhexidine hydrochloride, Triclosan, chlorhexidine dihydrochloride, chlorhexidine diacetate and combinations thereof. As an illustration of some suitable concentrations of various biocidal agents, Table 1 (shown below) contains the formulas of 12 representative formulations of various antimicrobial compositions (not in accordance with the invention as claimed) and shows the biocidal agent concentration in each formulation.

**TABLE 1**

| Formula | 1 | 2 | 3 | 4 | 5 | 6 | 7 | 8 | 9 | 10 | 11 | 12 |
|---|---|---|---|---|---|---|---|---|---|---|---|---|
| Modified Silicone | 0.1 | 0.1 | 0.2 | 0.1 | 0.5 | 0.1 | 0.1 | 0.5 | 0.1 | 0.1 | 0.1 | 0.0 |
| Isopropyl alcohol | 99.85 | 99.8 | 99.6 | 99.4 | 99.45 | 99.8 | 99.7 | 99.4 | 99.7 | 99.8 | 99.8 | 99.8 |
| Chlorhexidine Gluconate | 0.05 | 0.1 | 0.2 | 0.5 | | | | | | | | 0.2 |
| Chlorhexidine Diacetate | | | | | 0.05 | 0.1 | 0.2 | | | | | |
| Chloroxylenol | | | | | | | | 0.1 | 0.2 | | | |
| Triclosan | | | | | | | | | | 0.1 | | |
| Hexetidine | | | | | | | | | | | 0.1 | |

For instance, Table 1 shows that in formulations 1-4 and 12, chlorhexidine gluconate was selected as the biocidal agent and comprises from about 0.05% to about 0.5% of the antimicrobial composition's total weight. Table 1 further shows that in formulations 5, 6 and 7, chlorhexidine gluconate was selected as the biocidal agent and comprises from about 0.05% to about 0.2% of the antimicrobial composition's total weight. Table 1 further shows that in formulations 8 and 9, chloroxylenol comprises from about 0.1% to about 0.2% of the composition, by weight. Further still, Table 1 shows that in formulation 10, Triclosan comprises about 0.1% of the composition, by weight. Finally, Table 1 shows that in formulation 11, hexetidine comprises about 0.1% of the antimicrobial composition's total weight.

As mentioned, the antimicrobial composition also comprises a lubricant. The lubricant may include virtually any chemical or compound that is compatible with the teachings of the present invention. In general, a lubricant of the antimicrobial composition is compatible with the biocidal agent, and is biocompatible with medical devices. In particular, some embodiments of the present invention comprise a lubricant which is safe for use with medical devices used in infusion therapies or procedures.

In some embodiments, the antimicrobial composition employed in the present invention comprises a low viscosity silicone, such as a modified siloxane having a very low surface tension (i.e. about 20mN/m). In particular, some embodiments comprise a Silwet Surfactant (manufactured by Momentive, Inc.) as a lubricant.

The lubricant component of the antimicrobial composition may provide two or more functions to the composition. In some embodiments, a lubricant is provided to reduce friction between the surfaces of the medical device during assembly of the medical device. For example, in some embodiments a lubricant is provided to reduce friction between an inner surface geometry of a catheter device and the outer surface of a septum during assembly. Thus, the lubricant aids in insertion of the septum into the catheter adapter. The use of a lubricant may therefore reduce damage to the septum during assembly of the medical device.

The lubricant may also be provided to assist in binding the antimicrobial agent to various surfaces of the medical device during assembly. For example, in some embodiments a siloxane-based lubricant is incorporated into an antimicrobial composition which is applied to the surface of a silicon component of the medical device. The lubricant's chemical composition enhances the ability of the biocidal agent to remain bound to the silicon component of the medical device following evaporation of solvents within the antimicrobial composition. Thus, the siloxane chemical structure of the lubricant provides a matrix in which the biocidal agent is bound and held against the silicon component of the medical device. The lubricant is also effective in binding the biocidal agent to other surface or internal geometries of the medical device following evaporation of the composition's solvents.

As mentioned, the antimicrobial composition also comprises a carrier solvent. The carrier solvent may, in the present technology in general, comprise virtually any fluid that is capable of dissolving the biocidal agent and the lubricant of the antimicrobial composition. The solvent is further generally safe for medical device intended for use in infusion therapies and procedures. Some examples of such solvents may comprise alcohol, water, glycol, polypropylene glycol, polyol glycol, poloxamer, glycerin, and combinations thereof. Nevertheless, in some presently preferred embodiments, the solvent comprises a liquid that readily evaporates at room temperature and 1 atmosphere (e.g., has a relatively high vapor pressure). Additionally, in some presently preferred embodiments, the solvent has a relatively low viscosity such that the solvent may carry the biocidal agent and lubricant to various internal geometries and surfaces of a medical device by blown with air. Examples of such solvents may include alcohol and/or water.

However, in the invention as claimed, the carrier solvent comprises a non-alcohol based solvent in which the biocidal agent and lubricant are dissolved, and which readily undergoes evaporation at ambient conditions.

Indeed, in some aspects of the present technology, the solvent comprises one or more alcohols. In addition to acting as a solvent, the alcohol(s) may have several other advantageous characteristics. For example, the alcohol may further increase the effectiveness of the antimicrobial composition by acting as a biocidal agent that kills at least some of the pathogens it contacts. In another example, the alcohol evaporates relatively quickly thereby leaving or depositing the biocidal agent and lubricant on desired surfaces of a medical device. However, in the invention as claimed, the carrier solvent comprises a non-alcohol based solvent in which the biocidal agent and lubricant are dissolved, and which readily undergoes evaporation at ambient conditions.

While in the present technology in general the solvent can comprise any alcohol that is capable of dissolving the biocidal agent (preferably chlorhexidine gluconate) and the lubricant, in some aspects, the alcohol comprises a lower alcohol having from 1 to 6 carbon atoms. Some examples of such alcohols include methanol, ethanol, n-propanol, isopropanol, butanol, pentanol, hexanol, and so forth. In some presently preferred aspects of the present technology, however, the alcohol is selected from isopropanol and ethanol.

However, in the invention as claimed, the carrier solvent comprises a non-alcohol based solvent in which the biocidal agent and lubricant are dissolved, and which readily undergoes evaporation at ambient conditions.

In some aspects of the present technology in general, the solvent comprises more than one type of alcohol. In such embodiments, the antimicrobial composition may comprise any suitable number of alcohols, including 2, 3, 4, or more alcohols. Additionally, the solvent may comprise any suitable combination of alcohols. In one example, the solvent comprises isopropanol, butanol, and ethanol. In another example, the solvent comprises isopropanol and ethanol.

Where the solvent comprises more than one alcohol, the various alcohols may be present in the antimicrobial composition in any suitable ratio with respect to each other. For instance, in some aspects of the present technology where the antimicrobial composition comprises two alcohols (e.g., isopropanol and ethanol), the ratio of the first alcohol (e.g., isopropanol) to the second alcohol (e.g., ethanol) is from about 1:10 to about 1:1. In other aspects, the ratio of the first alcohol (e.g., isopropanol) to the second alcohol (e.g., ethanol) is from about 1:3 to about 1:1. In still other aspects, the ratio of the first alcohol (e.g., isopropanol) to the second alcohol (e.g., ethanol) is about 1:1.4 ± 0.2.

However, in the invention as claimed, the carrier solvent comprises a non-alcohol based solvent in which the biocidal agent and lubricant are dissolved, and which readily undergoes evaporation at ambient conditions.

Where the solvent in the antimicrobial composition comprises at least one alcohol, the antimicrobial composition may comprise any suitable amount of alcohol. In one example, one or more alcohols comprise at least 40% of the antimicrobial composition's total weight. In another example, alcohol comprises from about 40% to about 99% of the antimicrobial composition, by weight. In still another example, alcohol comprises from more than about 9% to about 99% of the antimicrobial composition. In yet another example, alcohol comprises from about 60% to about 80% of the antimicrobial composition, by weight. By way of example, formulations 1-12 in Table 1 (not according to the claimed invention), include isopropanol from about 99.0% to about 99.8% by weight of the composition. Indeed, in some presently preferred aspects, one or more alcohols (e.g., ethanol and isopropanol) account for about 70% ± 5% of the antimicrobial composition's overall weight.

However, in the invention as claimed, the carrier solvent comprises a non-alcohol based solvent in which the biocidal agent and lubricant are dissolved, and which readily undergoes evaporation at ambient conditions.

As mentioned above, in some embodiments, the solvent comprises water. In such embodiments, the water may be provided to the antimicrobial composition in any suitable aqueous solution, including a dilute alcohol or other solution containing water. Nevertheless, in some embodiments, the water comprises purified water, such as United States Pharacopeia ("USP") water or de-ionized water.

Where the antimicrobial composition comprises water, the composition may comprise any suitable amount of water. Indeed, in some embodiments not according to the present invention, in addition to the chlorhexidine gluconate, alcohol (i.e. carrier solvent), and/or any other suitable ingredient (i.e. lubricant), the remaining portion of the antimicrobial composition comprises water. In one example, the antimicrobial composition comprises from about 1% to about 99% water. For instance, water may account for more than 90% of the antimicrobial composition. In some embodiment, about 20% to about 40% the antimicrobial composition's overall weight is water. In still another example, the antimicrobial composition comprises from about 25% to about 30% water, by weight.

In some embodiments, the antimicrobial composition optionally comprises at least one other non-alcohol biocidal agent. In such embodiments, the second biocidal agent can comprise any suitable chemical or chemicals that kill, reduce, or otherwise impede pathogen proliferation while allowing the antimicrobial composition to sanitize surfaces. The second biocidal agent may further be selected based upon the biocidal agent leaving a tacky residue upon removal of a solvent from the antimicrobial composition. Some examples of suitable second biocidal agents include triclosan (5-chloro-2-(2,4-dichlorophenoxy)phenol)), silver and/or copper ions and nanoparticles (e.g., tinosan silver dihydrogen citrate), silver sulphadiazine, an imidozole, a triazole, an allyamine, phenol, hexachlorophene, an antibiotic, a sulfonamide, etc.

Where the antimicrobial composition comprises a second biocidal agent, the antimicrobial composition may comprise any suitable portion of the second biocidal agent. In one example, a second biocidal agent (e.g., triclosan) comprises from about 0.01% to about 10% of the total weight of the antimicrobial composition. In another example, the second biocidal agent comprises from about 0.1% to about 5% of the antimicrobial composition, by weight. In still another example, the second biocidal agent comprises from about 0.5% to about 2% of the antimicrobial composition, by weight.

In addition to the aforementioned ingredients, the antimicrobial composition may comprise any suitable ingredient, at any suitable concentration, which allows the antimicrobial composition to coat surfaces with the biocidal agent(s), be suitable for use in infusion therapies and/or procedures, and to dry with leaving a tacky residue. Some examples of such optional ingredients may comprise one or more known or novel tackifying agents, thickening agents, neutralizing agents, pH adjusters, metallic salts, dyes, fragrances, and/or other suitable chemicals.

The antimicrobial composition can also be modified to have any suitable characteristic. For example, while in some presently preferred embodiments, the antimicrobial composition comprises a liquid, in other embodiments, the antimicrobial composition can be modified to be a gel, a cream, a foam, or another fluid having a desired consistency/viscosity.

The antimicrobial composition may also be made in any suitable manner. For example, in some aspects the alcohol-based solvent and water components of the antimicrobial composition are mixed first, followed by the addition of other ingredients or components, in any order, at room temperature.

Generally, the antimicrobial compositions employed in the present invention are used to coat internal surfaces and/or internal geometries of medical devices. In particular, the antimicrobial compositions of the present invention are used: 1) to provide antimicrobial coating to internal surface of a medical device, and 2) to assist in the assembly of medical devices, wherein the properties of the antimicrobial compositions provide antimicrobial properties and optimize assembly of the medical device.

In some embodiments, an antimicrobial composition is provided which includes various components which leave a sticky or tacky residue following evaporation of solvents within the composition. As such, it may be undesirable to use some of the embodiments of the present invention for coating an external surface of a medical device. For example, it may be undesirable to apply some of the antimicrobial compositions of the present invention to a surface of a medical device which may come into contact with the skin or clothing of a user or patient. Thus, some of the antimicrobial compositions of the present invention are intended for application to internal surfaces or geometries of medical devices.

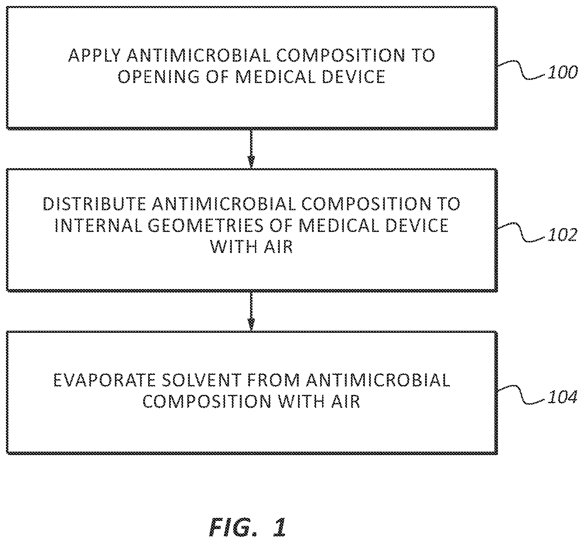

Referring now to Figure 1 (not in accordance with the invention as claimed), a method for applying an antimicrobial composition to a medical device, is shown. In some aspects of the present disclosure, a method is provided having a first step 100 whereby an antimicrobial composition is applied to an opening of a medical device. The liquid and viscous nature of the antimicrobial composition permits easy application of the composition to the opening of the medical device. For example, in some embodiments the antimicrobial composition is applied to the opening of the medical device via a liquid pump, whereby an aliquot of the antimicrobial composition is applied through the opening and into an internal portion of the medical device.

The antimicrobial composition is then distributed to the internal geometries of the medical device by blowing air through the opening of the medical device, as shown in step 102. For example, in some embodiments compressed air is directed into the opening of the medical device thereby distributing the antimicrobial composition across and through the various internal surfaces in geometries of the medical device. Accordingly, in some embodiments the medical device further comprises a second opening, or exit through which the air may escape. By blowing air through the opening of the medical device, the antimicrobial composition is distributed through the internal surfaces and geometries of the medical device thereby coating the surfaces with the antimicrobial composition. In some embodiments, excess antimicrobial composition is forced through the internal surfaces by the air and exits the device with the air through a second opening. Thus, the internal surfaces and geometries of the medical device comprise a thin and generally uniform layer of antimicrobial composition.

In the claimed invention, the process of blowing air through the opening of the medical device forces excess antimicrobial composition between opposing surfaces of components within the medical device. In the claimed invention, a septum is seated within a channel or groove formed on the inner surface of a catheter adapter. The process of blowing the antimicrobial composition through the medical device forces excess antimicrobial composition between the septum and the channel of the catheter adapter. Upon removal of the solvent from the antimicrobial composition (such as by evaporation), the portion of antimicrobial composition interposed between the septum and the catheter adapter forms a sticky or tacky residue which acts to adhere the outer surface of the septum to the inner surface of the catheter adapter. As such, the septum is secured and retained within its desired position via the tacky properties of the antimicrobial composition residue.

Following distribution of the antimicrobial composition, the flow of air through the medical device is continued to facilitate in evaporating the solvent of the antimicrobial composition, as shown in step 104. As the solvent evaporates, the biocidal agents and the lubricant of the antimicrobial composition adhere to the internal surfaces and geometries of the medical device, as discussed above.

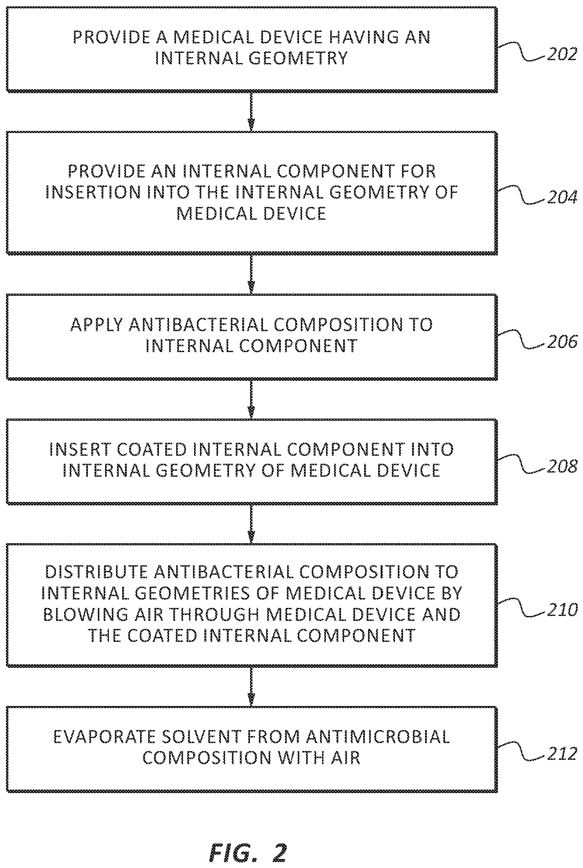

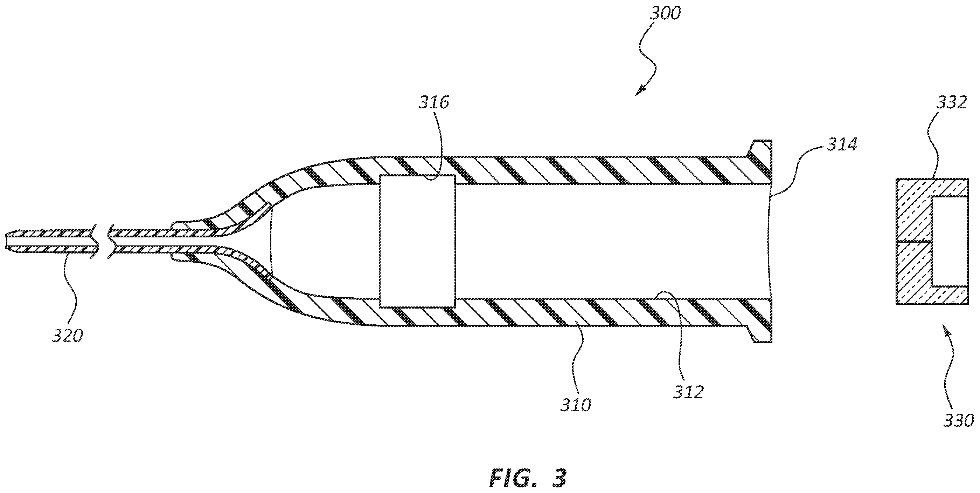

Referring now to Figure 2, a method for applying an antimicrobial composition to an internal surface or geometry of a medical device is shown. The method of Figure 2 is further shown diagrammatically in Figures 3-6, which will be discussed concurrently with Figure 2. In the present invention, t he claimed invention is obtainable by a method having a first step 202 whereby a medical device is provided having an internal geometry. A medical device of the present technology in general may include any device or component of a device for which an antimicrobial composition would provide a benefit. Further, in some aspects a medical device of the present invention may include any device or component of a device used in an infusion therapy or procedure. Non-limiting examples of medical devices or components which are compatible with the teachings of the present disclosure include catheters, catheter adapter's, needles, Luer adapters, intravenous tubing, syringes, trocars,
catheters, catheter adapter's, needles, Luer adapters, intravenous tubing, syringes, trocars, lancets, infusion pumps, septa, septum actuators, needle adapters, and various safety devices for use with intravenous catheter assemblies. In the invention as claimed, however, a catheter assembly having a catheter adapter operably coupled to a catheter is provided.

In some embodiments, a medical device further comprises an internal component which is inserted into an internal geometry of the medical device during assembly, as shown in step 204. With reference to Figure 3, a representative embodiment of a medical device 300 having one or more internal geometries and/or surfaces is shown. In the claimed invention, the medical device 300 comprises an intravenous catheter assembly having a catheter adapter 310 operably coupled to a catheter 320, wherein the catheter adapter 310 and catheter 320 each comprise various internal surfaces and geometries. In particular, in some embodiments catheter adapter 310 comprises an inner surface 312 which includes an annular channel 316. Annular channel 316 is sized and configured to receive an internal component 330 which provides a useful function to medical device 300. In the claimed invention, the internal component 330 comprises a septum.

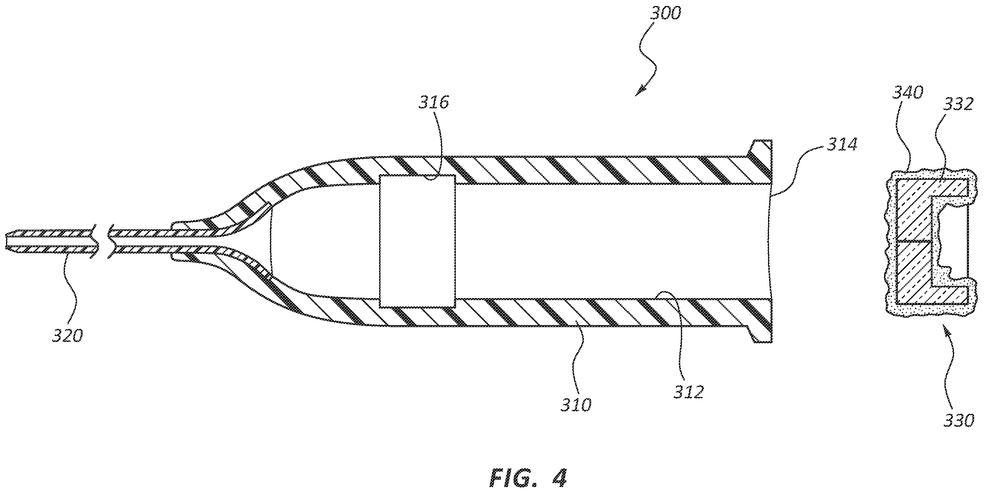

The process of assembling medical device 300 requires that internal component or septum 330 be inserted into a catheter adapter 310 and seated within annular channel 316. In some embodiments, septum 330 comprises an elastic, polymer material. Conversely, catheter adapter 310 comprises a rigid polymer material, such as a polycarbonate, polyurethane or polyethylene material. The physical properties of catheter adapter 310 and septum 330 provide friction between inner surface 312 of catheter adapter 310 and the outer surface 332 of septum 330. 1.. The outer diameter of septum 330 is greater than an opening 314 of catheter adapter 310. As such, septum 330 is required to compress inwardly or skew in shape during insertion of septum 330 through opening 314. This provides further pressure and friction between septum 330 and inner surface 312 of catheter adapter 310. Accordingly, the antimicrobial composition 340 is applied to outer surface 332 of septum 330 as a lubricant, prior to assembly, as shown in step 206 of Figure 2 and shown diagrammatically in Figure 4.

As discussed previously, the present invention employs an antimicrobial composition 340 which includes a lubricant component. The antimicrobial composition 340 further includes a biocidal agent and a non -alcohol based solvent.
Antimicrobial composition 340 may be applied to any outer or external surface of septum 330. Antimicrobial composition 340 provides at least one function as a lubricant to reduce friction between outer surface 332 and inner surface 312. Antimicrobial composition 340 further provides anti-pathogenic properties or functions to septum 330.

In some embodiments, the viscosity of antimicrobial composition 340 is selected such that composition 340 may be applied to outer surfaces 332 by dipping septum 330 into a container of antimicrobial composition 340. Further, in some embodiments antimicrobial composition 340 is applied to outer surfaces 332 by spraying. The viscosity of composition 340 enables collection of composition 340 on outer surfaces 332. For example, in some embodiments composition 340 is applied to outer surfaces 332 by dipping thereby resulting in the collection of a thin to moderate layer of composition 340 on outer surfaces 332. In other embodiments, antimicrobial composition 340 comprises a surfactant or other component which facilitates binding between composition 340 and outer surfaces 332. For example, in some embodiments a lubricant component of composition 340 comprises a low viscosity siloxane which is attracted to outer surface 332 of septum 330.

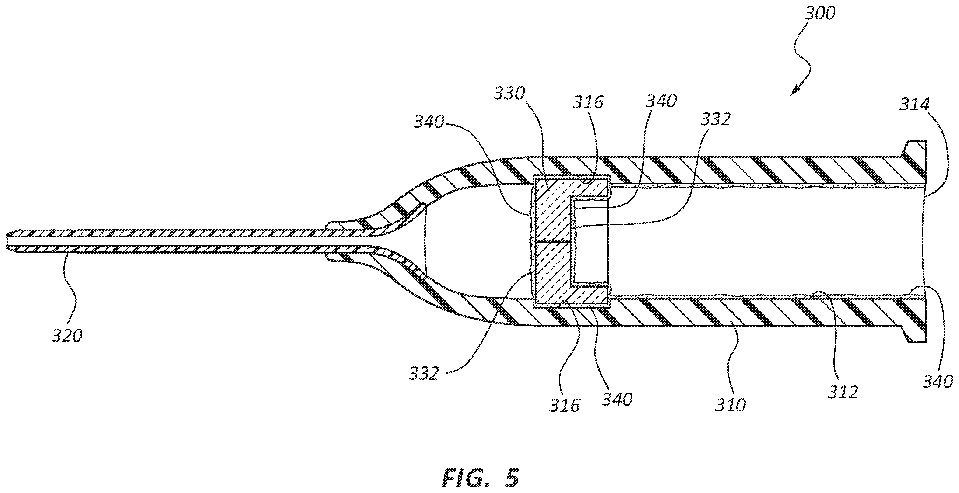

The coated septum 330 is then inserted into catheter adapter 310 through opening 314, as shown in step 208 of Figure 2, and shown diagrammatically in Figure 5. Septum 330 may be inserted into and advance distally within catheter adapter 310 by any compatible method. For example, in some embodiments septum 330 is inserted into catheter adapter 310 manually, such as by a probe (not shown). In other embodiments, septum 330 is inserted into catheter adapter 310 via air pressure. Septum 330 is advanced distally through the internal geometry of catheter adapter 310 until septum 330 is seated within channel 316. As shown, excess antimicrobial coating 340 is transferred from outer surface 332 to inner surface 312 during the assembly process. Further, a thin layer of antimicrobial coating 340 is interposed between outer surface 332 and channel 316.

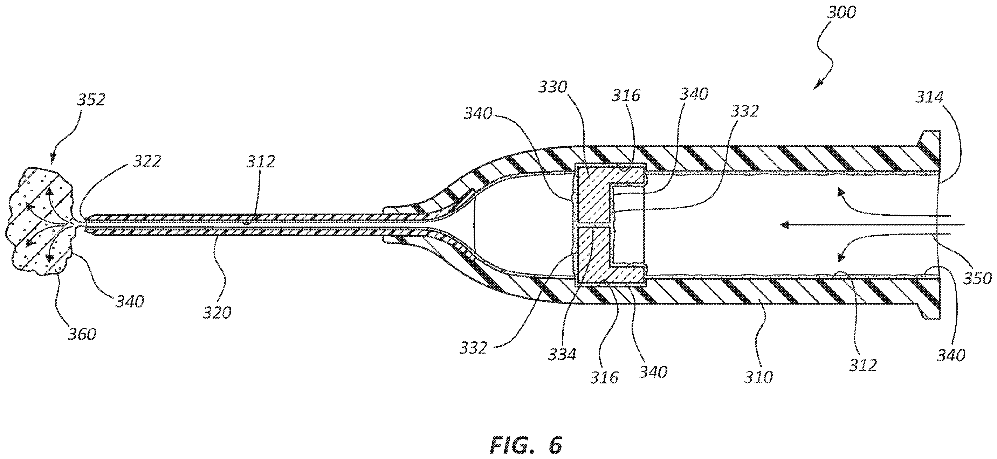

Antimicrobial composition 340 is distributed to the remaining internal geometries and surfaces 312 of catheter adapter and catheter 320 by blowing air 350 through opening 314 of catheter adapter 310, as shown in step 310 of Figure 2, and shown diagrammatically in Figure 6. Air 350 generally comprises compressed air that is applied directly to opening 314. The speed, pressure and flow rate of air 350 is selected to facilitate flow and effective distribution of composition 340 to internal surface 312 of catheter adapter 310 and catheter 320. Further, the speed, pressure and flow rate of air 350 is selected to facilitate flow and effective distribution of composition 340 to slit surfaces 334 of septum 330. Accordingly, the speed, pressure and flow rate of air 350 may be adjusted based upon the viscosity of antimicrobial composition 340.

Air 350 further facilitates evaporation of a solvent component 360 of antimicrobial composition 340. As discussed previously, some embodiments of antimicrobial composition 340 comprises an alcohol-based carrier solvent which facilitates flow and distribution of composition 340 to internal surfaces 312. Following the step of distributing antimicrobial composition 340, air 350 is further used to facilitate evaporation of carrier solvent 360 from antimicrobial composition 340, as shown in step 212, and further shown in Figure 6.

Compressed air 350 facilitates removal of excess antimicrobial composition 340 and evaporated solvent 360 which exits medical device 300 via opening 322 of catheter 320 as a fluid and vapor cloud 352. The removal of solvent vapor 360 condenses the remaining components of antimicrobial composition 340 onto internal surfaces and geometries 312 of catheter adapter 310 and catheter 320. Compressed air 350 further condenses the remaining components of composition 340 onto slit surfaces 334 of septum 330. In some embodiments, compressed air 350 removes solvent 360 from composition 340 interposed between outer surface 332 and channel 316. As such, a thin layer of composition remains interposed between the two surfaces.

As discussed previously, in some embodiments antimicrobial composition 340 comprises a biocidal agent that leaves a sticky or tacky residue upon evaporation of the composition's solvent. In other embodiments, antimicrobial composition 340 is modified to include an adhesive or other material which provides composition 340 with tacky or sticky properties following removal of the carrier solvent. Accordingly, in at least some embodiments of the present invention the portion of condensed antimicrobial composition interposed between septum 330 and channel 316 acts as an adhesive to bind and maintain the seated position of septum 330 within channel 316. The interposed portion of antimicrobial composition further acts to provide a seal between septum 330 and channel 316 thereby preventing blood and infusates from migrating between septum 330 and channel 316.

## Claims

1. A catheter assembly having a catheter adapter operably coupled to a catheter, wherein the catheter adapter and catheter each comprise various internal surfaces and geometries, said catheter assembly being obtainable from a method comprising the steps of:
providing a catheter assembly having an internal lumen;
providing a septum having an outer surface, wherein the outer diameter of the septum is greater than an opening of the catheter adapter;
coating the outer surface of the septum with an excess of an antimicrobial composition comprising:
a non-alcohol, biocidal agent;
a lubricant; and
a carrier solvent comprising a non-alcohol based solvent in which the biocidal agent and lubricant are dissolved, and which readily undergoes evaporation at ambient conditions;
inserting the coated septum into the internal lumen of the catheter adapter, wherein the septum compresses inwardly during insertion of the septum through the opening of the catheter adapter;
distributing the excess antimicrobial composition to the internal lumen by advancing and seating the coated septum within the internal lumen, such that it is seated within a channel or groove formed on the inner surface of the catheter adapter;
further distributing the excess antimicrobial composition within the internal lumen and forcing the excess antimicrobial composition between opposing surfaces of components within the catheter assembly by blowing air through the internal lumen and the seated septum; and
blowing additional air through the internal lumen and the seated septum to evaporate the carrier solvent of the antimicrobial composition thereby immobilizing the lubricant and the non-alcohol, biocidal agent within the internal lumen.

2. The catheter assembly of claim 1, wherein the non-alcohol, biocide agent is selected from the group consisting of phenol, quaternary ammonium, guanidine, pyridinium, benzalkonium, centrimide, hexetidine, benzethonium chloride, cetylpyridinium chloride, dequalium acetate, dequalinium chloride, chloroxylenol, chlorhexidine, chlorhexidine gluconate, chlorhexidine hydrochloride, Triclosan, chlorhexidine dihydrochloride, chlorhexidine diacetate and combinations thereof.

3. The catheter assembly of claim 1, wherein the non-alcohol, biocidal agent comprises from 0.01% to 1.0% of the antimicrobial composition, by weight, or wherein the non-alcohol, biocidal agent comprises from 0.01% to 0.5% of the antimicrobial composition, by weight.

4. The catheter assembly of any one of the preceding claims, wherein the lubricant has a surface tension of 20 mN/m.

5. The catheter assembly of claim 1, wherein the antimicrobial composition comprises:
a non-alcohol, biocidal agent comprising chlorhexidine gluconate, wherein the chlorhexidine gluconate comprises 0.01% to 0.5% of the antimicrobial composition, by weight;
a lubricant comprising a modified siloxane having a surface tension of 20 mN/m; and
a carrier solvent comprising a non-alcohol based solvent in which the biocidal agent and lubricant are dissolved, and which readily undergoes evaporation at ambient conditions.

6. The catheter assembly of claim 5, wherein the antimicrobial composition further comprises a second non-alcohol biocidal agent selected from the group consisting of phenol, quaternary ammonium, guanidine, pyridinium, benzalkonium, centrimide, hexetidine, benzethonium chloride, cetylpyridinium chloride, dequalium acetate, dequalinium chloride, chloroxylenol, chlorhexidine, chlorhexidine hydrochloride, Triclosan, chlorhexidine dihydrochloride, chlorhexidine diacetate and combinations thereof.

## Patentansprüche

1. Katheterbaugruppe, die einen Katheteradapter aufweist, der funktionell an einen Katheter gekoppelt ist, wobei der Katheteradapter und der Katheter jeweils verschiedene innere Oberflächen und Geometrien umfassen, wobei die Katheterbaugruppe durch ein Verfahren erhältlich ist, das die folgenden Schritte umfasst:
Bereitstellen einer Katheterbaugruppe mit einem inneren Lumen;
Bereitstellen eines Septums mit einer Außenfläche, wobei der äußere Durchmesser des Septums größer ist als eine Öffnung des Katheteradapters;
Beschichten der Außenfläche des Septums mit einem Überschuss einer antimikrobiellen Zusammensetzung, umfassend:
ein nichtalkoholisches Biozid;
ein Gleitmittel; und
ein Trägerlösungsmittel, das ein Lösungsmittel auf Nichtalkoholbasis umfasst, in dem das Biozid und das Gleitmittel gelöst sind, und das unter Umgebungsbedingungen leicht verdampft;
Einsetzen des beschichteten Septums in das innere Lumen des Katheteradapters, wobei das Septum während des Einsetzens des Septums durch die Öffnung des Katheteradapters nach innen zusammengedrückt wird;
Verteilen der überschüssigen antimikrobiellen Zusammensetzung auf das innere Lumen durch Vorschieben und Einsetzen des beschichteten Septums innerhalb des inneren Lumens, so dass es innerhalb eines Kanals oder einer Rille, die auf der inneren Fläche des Katheteradapters ausgebildet ist, sitzt;
weiter Verteilen der überschüssigen antimikrobiellen Zusammensetzung innerhalb des inneren Lumens und Zwingen der überschüssigen antimikrobiellen Zusammensetzung zwischen einander gegenüberliegende Flächen von Komponenten innerhalb der Katheterbaugruppe, indem man Luft durch das innere Lumen und das sitzende Septum bläst; und
Blasen zusätzlicher Luft durch das innere Lumen und das sitzende Septum, um das Trägerlösungsmittel der antimikrobiellen Zusammensetzung zu verdampfen, wodurch das Gleitmittel und das nichtalkoholische Biozid innerhalb des inneren Lumens immobilisiert werden.

2. Katheterbaugruppe gemäß Anspruch 1, wobei das nichtalkoholische Biozid aus der Gruppe ausgewählt ist, die aus Phenol, quartärem Ammonium, Guanidin, Pyridinium, Benzalkonium, Centrimid, Hexetidin, Benzethoniumchlorid, Cetylpyridiniumchlorid, Dequaliumacetat, Dequaliniumchlorid, Chlorxylenol, Chlorhexidin, Chlorhexidingluconat, Chlorhexidin-Hydrochlorid, Triclosan, Chlorhexidin-Dihydrochlorid, Chlorhexidindiacetat und Kombinationen davon besteht.

3. Katheterbaugruppe gemäß Anspruch 1, wobei das nichtalkoholische Biozid 0,01 bis 1,0 Gew.-% der antimikrobiellen Zusammensetzung ausmacht oder wobei das nichtalkoholische Biozid 0,01 bis 0,5 Gew.-% der antimikrobiellen Zusammensetzung ausmacht.

4. Katheterbaugruppe gemäß einem der vorstehenden Ansprüche, wobei das Gleitmittel eine Oberflächenspannung von 20 mN/m aufweist.

5. Katheterbaugruppe gemäß Anspruch 1, wobei die antimikrobielle Zusammensetzung umfasst:
ein nichtalkoholisches Biozid, das Chlorhexidingluconat umfasst, wobei das Chlorhexidingluconat 0,01 bis 0,5 Gew.-% der antimikrobiellen Zusammensetzung ausmacht;
ein Gleitmittel, das ein modifiziertes Siloxan mit einer Oberflächenspannung von 20 mN/m umfasst; und
ein Trägerlösungsmittel, das ein Lösungsmittel auf Nichtalkoholbasis umfasst, in dem das Biozid und das Gleitmittel gelöst sind, und das unter Umgebungsbedingungen leicht verdampft.

6. Katheterbaugruppe gemäß Anspruch 5, wobei die antimikrobielle Zusammensetzung weiterhin ein zweites nichtalkoholisches Biozid umfasst, das aus der Gruppe ausgewählt ist, die aus Phenol, quartärem Ammonium, Guanidin, Pyridinium, Benzalkonium, Centrimid, Hexetidin, Benzethoniumchlorid, Cetylpyridiniumchlorid, Dequaliumacetat, Dequaliniumchlorid, Chlorxylenol, Chlorhexidin, Chlorhexidin-Hydrochlorid, Triclosan, Chlorhexidin-Dihydrochlorid, Chlorhexidindiacetat und Kombinationen davon besteht.

## Revendications

1. Ensemble de cathéter comportant un adaptateur de cathéter couplé de manière opérationnelle à un cathéter, dans lequel l'adaptateur de cathéter et le cathéter comprennent chacun diverses surfaces internes et géométries, ledit ensemble de cathéter pouvant être obtenu par un procédé comprenant les étapes consistant à :
fournir un ensemble de cathéter ayant une lumière interne ;
fournir un septum ayant une surface externe, où le diamètre externe du septum est supérieur à une ouverture de l'adaptateur de cathéter ;
enduire la surface externe du septum avec un excès d'une composition antimicrobienne comprenant :
un agent biocide sans alcool ;
un lubrifiant ; et
un solvant support comprenant un solvant sans alcool dans lequel l'agent biocide et le lubrifiant sont dissous, et qui s'évapore facilement dans les conditions ambiantes ;
insérer le septum enduit dans la lumière interne de l'adaptateur de cathéter, où le septum se comprime vers l'intérieur durant son insertion par l'ouverture de l'adaptateur de cathéter ;
répartir la composition antimicrobienne en excès dans la lumière interne en avançant et en plaçant le septum enduit dans la lumière interne, de manière à ce qu'il soit placé dans un canal ou une rainure formé sur la surface interne de l'adaptateur de cathéter ;
répartir davantage la composition antimicrobienne en excès dans la lumière interne et la forcer entre les surfaces opposées des composants à l'intérieur de l'ensemble de cathéter en soufflant de l'air à travers la lumière interne et le septum ; et
souffler de l'air supplémentaire à travers la lumière interne et le septum pour faire évaporer le solvant support de la composition antimicrobienne, immobilisant ainsi le lubrifiant et l'agent biocide sans alcool à l'intérieur de la lumière interne.

2. Ensemble de cathéter selon la revendication 1, dans lequel l'agent biocide sans alcool est choisi dans le groupe constitué par le phénol, l'ammonium quaternaire, la guanidine, le pyridinium, le benzalkonium, le cétrimide, l'hexétidine, le chlorure de benzéthonium, le chlorure de cétylpyridinium, l'acétate de déqualinium, le chlorure de déqualinium, le chloroxylénol, la chlorhexidine, le gluconate de chlorhexidine, le chlorhydrate de chlorhexidine, le Triclosan, le dichlorhydrate de chlorhexidine, le diacétate de chlorhexidine et leurs combinaisons.

3. Ensemble de cathéter selon la revendication 1, dans lequel l'agent biocide sans alcool constitue de 0,01 % à 1,0 % de la composition antimicrobienne, en poids, ou dans lequel l'agent biocide sans alcool constitue de 0,01 % à 0,5 % de la composition antimicrobienne, en poids.

4. Ensemble de cathéter selon l'une quelconque des revendications précédentes, dans lequel le lubrifiant a une tension de surface de 20 mN/m.

5. Ensemble de cathéter selon la revendication 1, dans lequel la composition antimicrobienne comprend :
un agent biocide sans alcool comprenant du gluconate de chlorhexidine, où le gluconate de chlorhexidine constitue 0,01 % à 0,5 % de la composition antimicrobienne, en poids ;
un lubrifiant comprenant un siloxane modifié ayant une tension de surface de 20 mN/m ; et
un solvant support comprenant un solvant sans alcool dans lequel l'agent biocide et le lubrifiant sont dissous, et qui s'évapore facilement dans les conditions ambiantes.

6. Ensemble de cathéter selon la revendication 5, dans lequel la composition antimicrobienne comprend en outre un second agent biocide sans alcool choisi dans le groupe constitué par le phénol, l'ammonium quaternaire, la guanidine, le pyridinium, le benzalkonium, le cétrimide, l'hexétidine, le chlorure de benzéthonium, le chlorure de cétylpyridinium, l'acétate de déqualinium, le chlorure de déqualinium, le chloroxylénol, la chlorhexidine, le chlorhydrate de chlorhexidine, le Triclosan, le dichlorhydrate de chlorhexidine, le diacétate de chlorhexidine et leurs combinaisons.
